# EUROPÄISCHE PATENTSCHRIFT

(11) **EP 2 057 126 B1**
(45) Veröffentlichungstag und Bekanntmachung des Hinweises auf die Patenterteilung: **10.11.2010**
(21) Anmeldenummer: 07801808.2
(22) Anmeldetag: 22.08.2007
(51) Int. Cl.: C07D 231/14

(54) **VERFAHREN ZUM HERSTELLEN VON 3-DIHALOMETHYL-PYRAZOL-4-CARBONSÄUREDERIVATEN**
PROCESS FOR PREPARING 3-DIHALOMETHYLPYRAZOLE-4-CARBOXYLIC ACID DERIVATIVES
PROCÉDÉ DE FABRICATION DE DÉRIVÉS D'ACIDE 3-DIHALOGÉNOMÉTHYL-PYRAZOL-4-CARBOXYLIQUE

(30) Priorität: 25.08.2006 DE 102006039909
(43) Veröffentlichungstag der Anmeldung: 13.05.2009
(73) Patentinhaber: Bayer CropScience AG, 40789 Monheim (DE)
(72) Erfinder: PAZENOK, Sergii, 42699 Solingen (DE); NORBERT, Lui, 51519 Odenthal (DE); NEEFF, Arnd, 51399 Burscheid (DE)
(86) Internationale Anmeldenummer: PCT/EP2007/007377
(87) Internationale Veröffentlichungsnummer: WO 2008/022777

(56) Entgegenhaltungen:
- WO-A-03/074491
- WO-A-2005/042468
- WO-A-2006/045504
- DATABASE CA [Online] CHEMICAL ABSTRACTS SERVICE, COLUMBUS, OHIO, US; PASHKEVICH, K. I. ET AL: "Reaction of 2-acetyl-substituted polyfluorinated .beta.-keto esters with amines" XP002469504 gefunden im STN Database accession no. 1989:114750 & IZVESTIYA AKADEMII NAUK SSSR, SERIYA KHIMICHESKAYA , (6), 1367-71 CODEN: IASKA6; ISSN: 0002-3353, 1988,

## Beschreibung

Die Erfindung betrifft ein Verfahren zum Herstellen von 3-Dihalomethyl-pyrazol-4-carbonsäurederivaten der Formel (I) durch Umsetzung von α-Fluoraminen der Formel (III) in Gegenwart von Lewissäuren mit Acrylsäurederivaten der Formel (II) zu Vinamidiniumsalzen der Formel (IV) und deren anschließender Umsetzung mit Hydrazinen, sowie die Vinamidiniumsalzen der Formel (IV) selbst.

3-Difluormethyl-pyrazol-4-carbonsäureester, -amide und -nitrile sind wichtige Synthesebausteine zur Herstellung von Agrowirkstoffen, insbesondere zur Herstellung von Pyrazolylcarboxanilid-Fungiziden.

WO-A-05 042 468 lehrt ein Verfahren zum Herstellen von 2-Dihaloacyl-3-aminoacrylsäureestern durch Umsetzung von Säurehalogeniden mit Dialkylaminoacrylsäureestern, sowie deren Umsetzung mit Hydrazinderivaten zu 3-Dihalogenmethyl-pyrazol-4-carbonsäureestern.

WO-A-03 051 820 lehrt die Herstellung von 2-Halogenacyl-3-aminoacrylsäureestern durch Umsetzung von N-substituierten 3-Aminoacrylsäureestern mit Halogenalkylcarbonsäureanhydriden und deren anschließende Umsetzung mit Hydrazinderivaten zu 3-Halogenalkylpyrazol-4-carbonsäureestern. Die Reaktion zu den 3-Halogenalkyl-pyrazol-4-carbonsäureestern verläuft bei Raumtemperatur unselektiv und muss daher bei tiefen Temperaturen (-80°C) durchgeführt werden.

WO-A-06 005 612 lehrt ein Verfahren zur Herstellung von 4,4-Difluor-3-oxo-buttersäureethylester durch Umsetzung von 2,2-Difluor-N-dialkyl-acetamid mit Essigsäureestern in Gegenwart von Basen. Der 4,4-Difluor-3-oxo-buttersäureethylester wird anschließend, wie in JACS, 73, 3684 (1951) beschrieben, mit Orthoameisensäuretrimethylester und Essigsäureanhydrid zum Ethyl-(2-ethoxymethylen)-4,4-difluoromethylacetoacetat umgesetzt, welches, gemäß der US-A-5,489,624, mit Methylhydrazin zum 3-Difluormethyl-1-methyl-4-pyrazolcarbonsäureethylester überführt werden kann. Die beschriebene Route beinhaltet zum einen eine Vielzahl von Reaktionsschritte, zum anderen ist das verwendete 2,2-Difluor-N-dialkyl-acetamid nicht kommerziell zugänglich und kann nur in geringen Ausbeuten von ca. 70 % durch Fluorierung von 2,2-Dichlor-N-dialkyl-acetamid erhalten werden.

Die im Stand der Technik vorbeschriebenen Verfahren weisen den Nachteil auf, dass die verwendeten Carbonsäurehalogenide, Halogenalkylcarbonsäureanhydride und Haloacrylester teuer sind, Korrosionsprobleme verursachen und/oder nur mit großem technischem Aufwand gereinigt werden können.

Der vorliegenden Erfindung liegt daher die Aufgabe zugrunde ein einfacheres und wirtschaftlicheres Verfahren zur Herstellung von 2-Halogenacyl-3-aminoacrylsäurederivaten, insbesondere von Estern, Nitrilen und Amiden, zur Verfügung zu stellen.

Die zuvor beschriebene Aufgabe wurde erfindungsgemäß gelöst durch ein Verfahren zum Herstellen von 3-Dihalogenmethyl-pyrazol-4-carbonsäurederivaten der Formel (I) 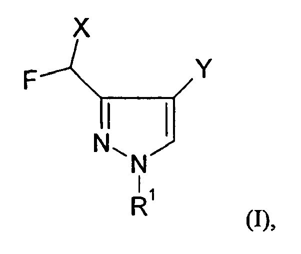 in welcher
- R¹: ausgewählt ist aus Wasserstoff, C₁₋₁₂-Alkylresten, C₅₋₁₈-Aryl- oder C₇₋₁₉-Arylalkyl- resten,
- Y: ausgewählt ist aus (C=O)OR⁶, CN und (C=O)NR⁷R^{8,} wobei R⁶ R⁷ und R⁸ unab- hängig voneinander ausgewählt sind aus C₁₋₁₂-Alkylresten, C₅₋₈-Aryl- oder C₇₋₁₉- Arylalkylresten; und
- X: F, Cl oder CF₃ ist
durch Umsetzung von α-Fluoraminen der Formel (III) 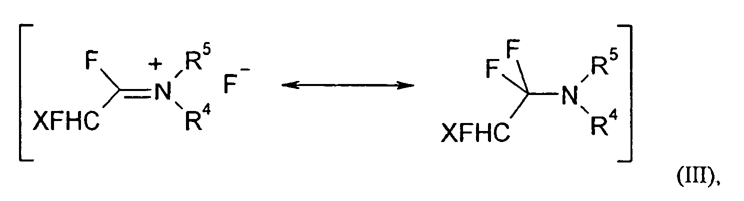 in welcher
- R⁴: ausgewählt ist aus C₁₋₁₂-Alkylresten, C₅₋₁₈-Aryl- oder C₇₋₁₉-Arylalkylresten,
- R⁵: unabhängig von R⁴ ausgewählt ist aus C₁₋₁₂-Alkylresten, C₅₋₁₈-Aryl- oder C₇₋₁₉- Arylalkylresten, und
- X: F, Cl oder CF₃ ist,
in Gegenwart von Lewissäuren (Z) mit Acrylsäurederivaten der Formel (II) 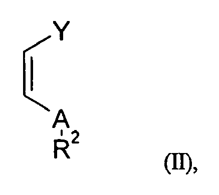 in welcher
- A: ausgewählt ist aus O, S und NR³,
- Y: ausgewählt ist aus (C=O)OR⁶, CN und (C=O)NR⁷R^{8,} wobei R⁶ R⁷ und R⁸ unabhängig voneinander ausgewählt sind aus C₁₋₁₂-Alkylresten, C₅₋₁₈-Aryl- oder C₇₋₁₉-Arylatkylresten; und
- R² und R³: unabhängig voneinander ausgewählt sind aus C₁₋₁₂-Alkylresten, C₅₋₁₈-Aryl- oder C₇₋₁₉-Arylalkylresten, -OR', -SR', -NR'₂ , wobei R' ein (C₁-C₅) Alkylrest sein kann,
oder R² und R³ gemeinsam mit dem N-Atom, an das sie gebunden sind einen fünf- oder sechsgliedrigen Ring bilden können,
und anschließender Umsetzung mit Hydrazinen gemäß der allgemeinen Formel (V)

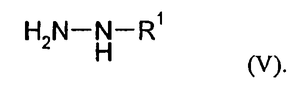

Ein weiterer Gegenstand der vorliegenden Erfindung betrifft die bei dem erfindungsgemäßen Verfahren als Zwischenprodukte entstehenden Vinamidiniumsalz der Formel (IV) 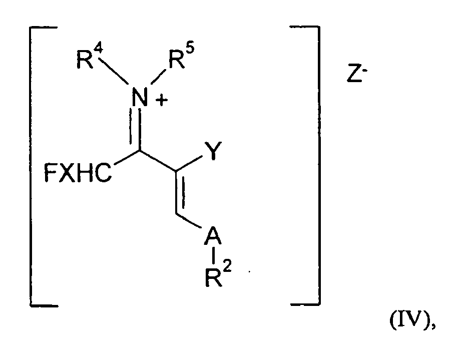 in welcher
alle Reste die zuvor genannten Bedeutungen haben.

Weitere Ausführungsformen der vorliegenden Erfindung können den abhängigen Ansprüchen und der Beschreibung entnommen werden.

Das erfindungsgemäße Verfahren kann anhand des folgenden Schema (I) erläutert werden:

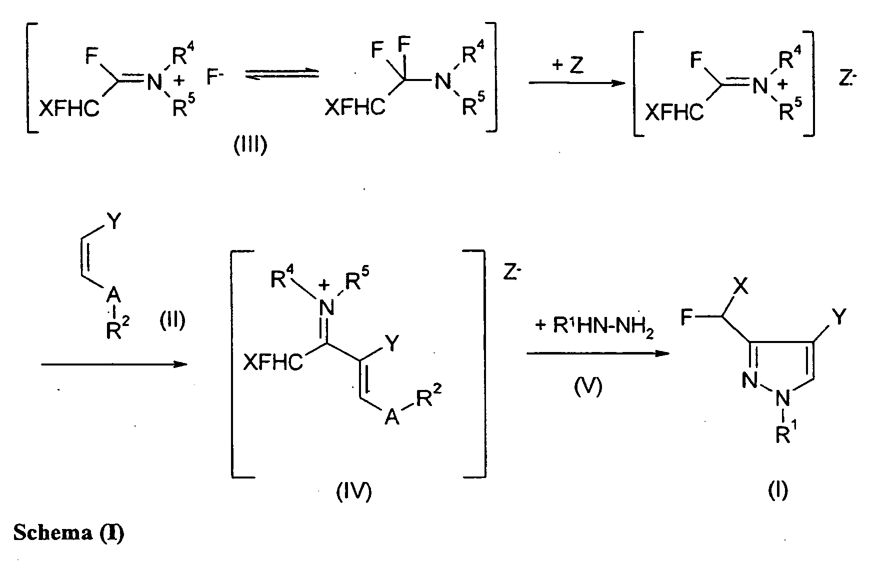

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Im Zusammenhang mit der vorliegenden Erfindung bezeichnet die Gruppe -X ein Halogenatom, das ausgewählt ist aus Fluor, Chlor, Brom und Iod, vorzugsweise Fluor, Chlor und Brom, besonders bevorzugt aus Fluor und Chlor.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CON-R₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für einen ArylGruppe mit 5 bis 18 Atomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome. Nicht umfasst sind solche Kombinationen, die den Naturgesetzen widersprechen und die der Fachmann daher, aufgrund seines Fachwissens, ausgeschlossen hätte. Beispielsweise sind Ringstrukturen mit drei oder mehreren benachbarten O-Atomen ausgeschlossen.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Acrylsäurederivate

Die gemäß der vorliegenden Erfindung verwendeten Acrylsäurederivate sind Verbindungen gemäß der allgemeinen Formel (II).

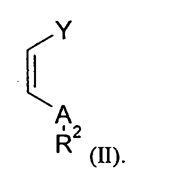

Bei dieser ist A ausgewählt aus O, S und NR³ und die Reste R² und R³ sind unabhängig voneinander ausgewählt aus C₁₋₁₂-Alkylresten, C₅₋₁₈-Aryl-, C₇₋₁₉-Arylalkylresten, Alkoxy-Gruppen (-OR'), Mercapto-Gruppen (-SR'), Amino-Gruppen (-NR'₂) , wobei R' ein C₁₋₅-Alkylrest sein kann.

Alternativ können R² und R³ gemeinsam mit dem N-Atom, an das sie gebunden sind einen fünf- oder sechsgliedrigen Ring bilden.

Vorzugsweise sind die Reste R² und R³ unabhängig voneinander ausgewählt aus C₂₋₈-Alkylresten, O-(C₂₋₆-Alkyl), S-(C₂₋₆-Alkyl), N(C₂₋₆-Alkyl)₂.

Besonders bevorzugt sind die Reste R² und R³ unabhängig voneinander ausgewählt aus C₃₋₆-Alkylresten, O-(C₃₋₄-Alkyl), S-(C₃₋₄-Alkyl), N(C₃₋₄-Alkyl)₂.

Im Zusammenhang mit der vorliegenden Erfindung bevorzugte Dialkylaminoacrylsäurederivate sind in den folgenden Formeln (II-a) bis (II-e) dargestellt.

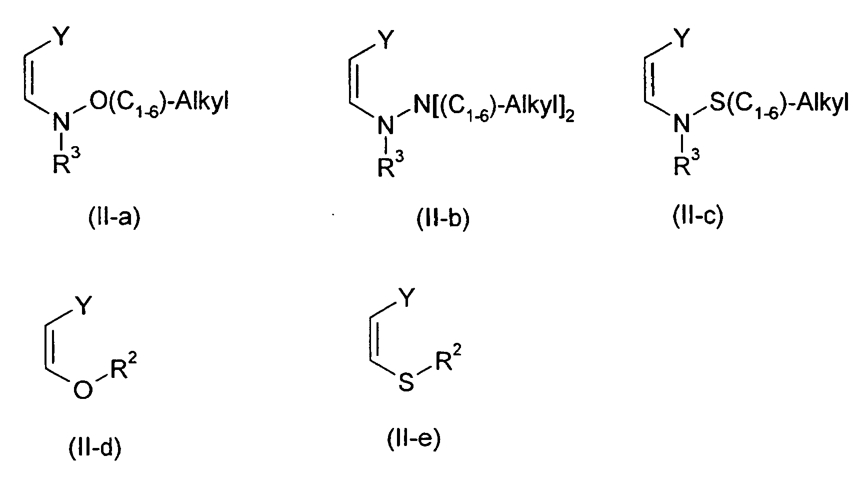

Die Gruppe Y ist ausgewählt aus Carbonsäureestergruppen ((C=O)OR⁶), Nitrilgruppen (CN) und Amidgruppen ((C=O)NR⁷R⁸), darin sind R⁶ R⁷ und R⁸ unabhängig voneinander ausgewählt aus C₁₋₁₂-Alkylresten, C₅₋₁₈-Aryl- oder C₇₋₁₉-Arylalkylresten; vorzugsweise aus C₂₋₈-Alkylresten, besonders bevorzugt aus C₃₋₆-Alkylresten.

Beispiele für erfindungsgemäß geeignete Acrylsäureester sind Methoxyacrylsäureester, Alkylthioacrylsäureester, 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid wobei 3-(N,N-Diethylamino)-acrylsäureethylester besonders bevorzugt ist.

Verfahren zur Herstellung von Dialkylaminoacrylsäureestern sind im Stand der Technik, beispielsweise in EP-A-0 608 725, vorbeschrieben.

Verfahren zur Herstellung von Dialkylaminoacrylnitrilen sind im Stand der Technik, beispielsweise von Rene et al in Synthesis (1986), (5), 419-420 beschrieben.

Die Acrylsäurederivate können, falls notwendig, beispielsweise destillativ gereinigt werden. Dies ist jedoch im Allgemeinen im Zusammenhang mit der erfindungsgemäßen Reaktion nicht erforderlich.

### α-Fluoramine

Die gemäß der vorliegenden Erfindung verwendeten α-Fluoramine sind Verbindungen gemäß der allgemeinen Formel (III) 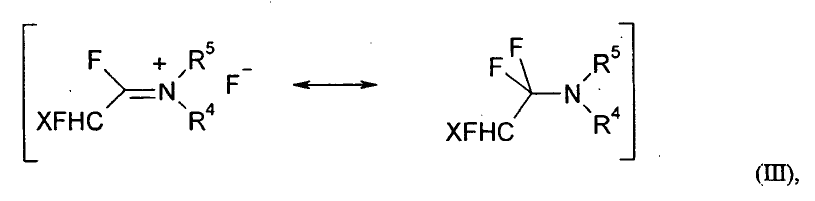 in welcher
- R⁴: ausgewählt ist aus C₁₋₁₂-Alkylresten, C₅₋₁₈-Aryl- oder C₇₋₁₉-Arylalkylresten, vorzugsweise aus C₂₋₈-Alkylresten, besonders bevorzugt aus C₃₋₆-Alkylresten.
- R⁵: unabhängig von R⁴ ausgewählt ist aus C₁₋₁₂-Alkylresten, C₅₋₁₈-Aryl- oder C₇₋₁₉- Arylalkylresten, vorzugsweise aus C₂₋₈-Alkylresten, besonders bevorzugt aus C₃₋₆- Alkylresten.
- X: CF₃, F oder Cl ist.

Die Verbindungen sind gemäß Petrov et al in Journal of Fluorine Chemistry 109 (2001) 25-31 und Dmowski et al in Chemistry of Organic Fluorine Compounds II, A Critical Review, ACS, Washington DC (1995) 263 durch Umsetzung von fluorierten/halogenierten Alkenen mit sekundären Aminen zugänglich und werden kommerziell beispielsweise durch DuPont vertrieben.

Die gemäß der vorliegenden Erfindung bevorzugt eingesetzten α-Fluoramine sind beispielsweise ausgewählt aus der Gruppe bestehend aus 1,1,2,2-Tetrafluorethyl-N,N-dimethylamin, 1,1,2,2--Tetrafluorethyl-N,N-diethylamin, 1,1,2-Trifluor-2-(trifluormethyl)ethyl-N,N-dimethylamin, 1,1,2-Trifluor-2-(trifluormethyl)ethyl-N,N-diethylamin (Ishikawa-Reagenz), 1,1,2-Trifluor-2-chlor-ethyl-N,N-dimethylamin und 1,1,2-Trifluor-2-chlor-ethyl-N,N-diethylamin (Yarovenko-Reagenz), wobei 1,1,2,2-Tetrafluorethyl-N,N-dimethylamin und 1,1,2,2-Tetrafluorethyl-N,N-diethylamin bevorzugt und 1,1,2,2-Tetrafluorethyl-N,N-dimethylamin besonders bevorzugt ist.

### Lewissäuren

Die zuvor beschriebenen α-Fluoramine reagieren in Gegenwart von Lewissäuren (Z) zu Immoniumsalzen, wie von Wakselman et al in J.C.S. Chem. Comm. 565 (1975) 956 beschrieben wurde.

Die Umsetzung der α-Fluoramine mit den Lewissäuren erfolgt vorzugsweise bei Temperaturen von -80 bis 50°C, vorzugsweise von -40 bis 40°C, besonders bevorzugt von 0 bis 30°C.

Gegebenenfalls kann auf den Zusatz einer Lewissäure verzichtet werden.

Die Reaktion kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus aliphatischen und aromatischen Kohlenwasserstoffen, wie z.B. n-Hexan, Benzol oder Toluol, die durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol oder THF; Nitrilen wie Methylnitril, Butylnitril oder Phenylnitril, wobei Dichlormethan und Acetonitril besonders bevorzugt sind.

Als Lewissäuren sind beispielsweise Verbindungen geeignet, die ausgewählt sind aus der Gruppe bestehend aus BF₃, AlCl₃, AlF₃, ZnCl₂, PF₅, SbF₅, SnCl₄, BiCl₃, GaCl₃, SiCl₄

Die Lewissäure und das α-Fluoramin werden vorzugsweise in äquimolaren Mengen eingesetzt. Alternativ kann die Lewissäure auch im Überschuss eingesetzt werden. Das Verhältnis von Lewissäure : α-Fluoramin liegt erfindungsgemäß zwischen 1:1 und 10:1, vorzugsweise zwischen 1:1 1 und 5:1, besonders bevorzugt zwischen 1:1 1 und 1:1.3.

In einer bevorzugten Ausführungsform des erfindungsgemäßen Verfahrens wird das α-Fluoramin in Substanz oder gelöst in einem geeigneten Lösungsmittel vorgelegt und sukzessive mit der Lewissäure versetzt.

Aufgrund der Hydrolyseempfindlichkeit der α-Fluoramine ist die Umsetzung des α-Fluoramins mit der Lewissäure in wasserfreien Apparaturen unter Inertgasatmosphäre durchzuführen.

Die erhaltenen Vinamidiniumsalze der Formel (IV) sind hingegen weder hygroskopisch noch hydrolyseempfindlich und können daher an der Luft gehandhabt und gelagert werden.

Die weitere Umsetzung der Immoniumsalze mit den Dialkylaminoacrylsäureestern der Formel (II) erfolgt vorzugsweise ohne vorherige Isolierung der Immoniumsalze. In einer weiteren erfindungsgemäßen Ausführungsform können die Immoniumsalze zuvor isoliert und nach Bedarf eingesetzt werden.

Die Umsetzung der Immoniumsalze mit den Acrylsäurederivaten der Formel (II) zu den Vinamidiniumsalzen der Formel (IV) 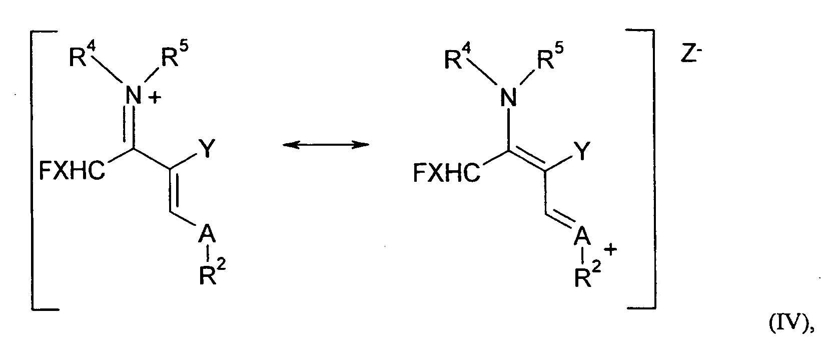 wobei das Anion Z' beispielsweise ausgewählt ist aus der Gruppe bestehend aus [BF₄]⁻, [AlCl₃F]⁻, [AlF₄]⁻, [ZnCl₂F]⁻, [PF₆]⁻, [SbF₆]⁻, [SnCl₄F]⁻, [BiCl₃F]⁻, [GaCl₃F]⁻, [ZnCl₂F]⁻, [SnCl₄F]⁻, [BiCl₃F]⁻, [GaCl₃F]⁻, [SiCl₄F]⁻ kann bei Temperaturen von -40 bis 60°C, vorzugsweise von -20 bis 40°C, besonders bevorzugt von 0 bis 50°C erfolgen.

Die Immoniumsalze und die Acrylsäurederivate werden vorzugsweise in äquimolaren Mengen eingesetzt. Alternativ können die Immoniumsalze oder die Acrylsäurederivate auch im Überschuss eingesetzt werden. Das Verhältnis von Immoniumsalz : Acrylsäurederivat liegt erfindungsgemäß zwischen 1:10 und 10:1, vorzugsweise zwischen 1:5 und 5:1, besonders bevorzugt zwischen 1.3:1 und 1:1.3.

Als Lösungsmittel werden vorzugsweise diejenigen Lösungsmittel verwendet, die auch zuvor für die Synthese der Immoniumsalze eingesetzt wurden.

In einer bevorzugten Ausführungsform des erfindungsgemäßen Verfahrens wird das Immoniumsalz in Substanz oder gelöst in einem geeigneten Lösungsmittel vorgelegt und sukzessive mit dem Acrylsäurederivate versetzt.

In einer weiteren Ausführungsform des erfindungsgemäßen Verfahrens werden Acrylsäurederivate (II) und α-Fluoramine (III) ggf. in einem Lösungsmittel vorgelegt und sukzessive mit der Lewissäure versetzt. Abschließend wird dann ein Hydrazin der Formel (V) zugesetzt.

Die Immoniumsalze der Formel (IV) können durch einfaches Entfernen des Lösungsmittels isoliert werden.

Vorzugsweise werden die Immoniumsälze der Formel (IV) jedoch ohne vorherige Isolierung mit Hydrazinen der allgemeinen Formel (V), vorzugsweise mit Methylhydrazin, zu 3-Dihalogenmethyl-pyrazol-4-carbonsäureestern der Formel (I) umgesetzt.

Ganz besonders bevorzugt werden Hydrazin, Methylhydrazin und Ethylhydrazin eingesetzt, wobei Methylhydrazin noch weiter bevorzugt ist.

Als bevorzugte Verbindungen der allgemeinen Formel (I) seien genannt:
1-Methyl-3-difluormethyl-4-pyrazolcarbonsäuremethylester, 1-Methyl-3-difluormethyl-4-pyrazolcarbonsäureethylester, 1-Methyl-3-chlorfluormethyl-4-pyrazolcarbonsäuremethylester, 1-Methyl-3-chlorfluormethyl-4-pyrazolcarbonsäureethylester, 1-Methyl-3-(trifluormethyl)fluormethyl-4-pyrazolcarbonsäuremethylester,1-Methyl-3-(trifluormethyl)fluormethyl-4-pyrazolcarbonsäureethylester, wobei 1-Methyl-3-difluormethyl-4-pyrazolcarbonsäureethylester und 1-Methyl-3-difluormethyl-4-pyrazolcarbonsäuremethylester besonders bevorzugt sind.

Bevorzugt wird die Umsetzung der Immoniumsalze der Formel (IV) mit den Hydrazinen der Formel (V) in Gegenwart von Lösungsmitteln durchgeführt. Geeignete Lösungsmittel sind beispielsweise diejenigen, die auch für die Durchführung der vorherigen Schritte angegeben wurden.

Die Umsetzung mit den Alkylhydrazinen kann beispielsweise und bevorzugt bei -30 bis +80°C, besonders bevorzugt bei -20 bis +25°C und ganz besonders bevorzugt bei -10 bis +40°C erfolgen.

Aus Gründen der Wirtschaftlichkeit wird die Durchführung bei Raumtemperatur (RT) bevorzugt.

Als besonders vorteilhaft kann herausgestellt werden, dass die Bildung der 3-Halogenalkyl-4-pyrazolcarbonsäurederivate auch bei Raumtemperatur unter hoher Regioselektivität verläuft.

Die in geringem Anteil (≤ 8%) entstehenden regioisomeren 4-Halogenalkyl-3-pyrazolcarbonsäurederivate können von den gewünschten Produkten aufgrund ihrer unterschiedlichen physikalischen Eigenschaften durch geeignete Verfahren, wie z.B. Destillation oder Kristallisation oder durch einfache Wäsche mit z.B Cyclohexane, abgetrennt werden.

Weiterhin ist als vorteilhaft zu erwähnen, dass alle Reaktionsschritte des erfindungsgemäßen Verfahrens, ohne zwischenzeitliche Aufreinigung/Isolation der Zwischenstufen/Zwischenprodukte, nacheinander durchgeführt werden können.

Die 3-Halogenalkyl-4-pyrazolcarbonsäurederivate der Formel (I) können gegebenenfalls in an sich bekannter Weise (Houben-Weyl, Methoden der organischen Chemie, 4. Auflage, Band E5, S 223ff.) beispielsweise durch saure oder alkalische Verseifung in 3-Halogenalkyl-4-pyrazolcarbonsäuren überführt werden.

Bevorzugt ist die alkalische Verseifung. Diese kann in an sich bekannter Weise beispielsweise durch Umsetzung mit Basen wie z.B. Alkalimetallhydroxiden wie z.B. Lithium-, Natrium- oder Kaliumhydroxid oder deren wässrige Lösungen erfolgen. Als Lösungsmittel sind beispielsweise Wasser, Alkohole wie z.B. Methanol, Ethanol und Isopropanol, aromatische Kohlenwasserstoffe wie z.B. Toluol, Aceton, Pyridin oder Mischungen solcher Lösungsmittel geeignet.

Im Zusammenhang mit der vorliegenden Erfindung bevorzugte 3-Halogenalkyl-4-pyrazolcarbonsäuren sind 1-Methyl-3-difluormethyl-4-pyrazolcarbonsäure, 1-Methyl-3-chlorfluormethyl-4-pyrazolcarbonsäure, 1-Methyl-3-(trifluromethyl)fluormethyl-4-pyrazolcarbonsäure, 3-Difluormethyl-4-pyrazolcarbonsäure, 3-(Trifluromethyl)fluormethyl-4-pyrazolcarbonsäure und 3-Chlorfluormethyl-4-pyrazolcarbonsäure, wobei 1-Methyl-3-difluormethyl-4-pyrazolcarbonsäure besonders bevorzugt ist.

Die Erfindung soll anhand der folgenden Ausführungsbeispiele näher erläutert werden, ohne sie jedoch auf diese zu beschränken.

### Beispiele

### Beispiel 1:

### N-[(2E)-1-(difluormethyl)-3-(dimethylamino)-2-(ethoxycarbonyl)prop2-en-1-yliden]-N-methylmethanaminium-tetrafluoroborat

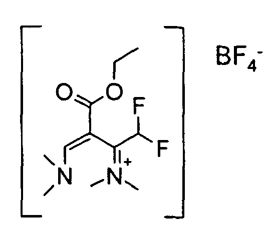

8,8 g (60 mmol) N-1,1,2,2-Tetrafluorethyldimethylamin wurden in 50 ml Dichlormethan unter Argon vorgelegt und 8,2 g (60 mmol) Bortrifluoriddiethylether Komplex bei RT zugegeben. Das Gemisch wurde 30 min gerührt und dann mit 7,15 g (50 mmol) Dimethylaminoacrylsäureethylester versetzt. Nach 2h Rühren bei RT und Entfernen des Dichlormethans im Vakuum wurden 12,4 g des Produktes (100 % Ausbeute) als gelbes Öl erhalten.

**¹⁹F-NMR** (CDCl₃) δ=-120,35, (d, 2F, J=51 Hz); -151,2 (s, 4F) ppm.

**¹H-NMR** (CDCl₃) δ=1,25 (t, 3H); 2,8, (s, 6H), 3,45 (m,6H); 4,2 (qu, CH₂); 6,87 (t, 1H); 8,16 (s, 1 H) ppm.

### Beispiel 2:

### N-[(2E)-1-(Chlorfluormethyl)-3-(dimethylamino)-2-(ethoxycarbonyl)-prop-2-en-1-yliden]-N-methylmethanaminiumtetrafluoroborat

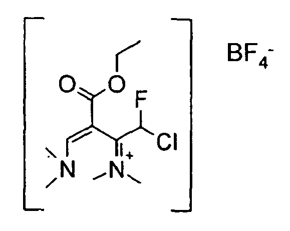

17,6 g (0,1 mmol) N-1,1,2-Trifluoro-2-chloroethyldimethylamin wurden in 100 ml Dichlormethan unter Argon vorgelegt und bei RT mit 13,6 g (0,1 mol) Bortrifluoriddiethylether Komplex versetzt. Nach 30 min Rühren wurden 14,3 g (0,1 mmol) Dimethylaminoacrylsäureethylester zugegeben und 2 h bei RT gerührt. Nach dem Entfernen des Dichlormethan im Vakuum erhielt man 25,2 g (95 % d.T) des Produkts.

### Beispiel 3:

### Ethyl-3-(difluoromethyl)-1-methyl-1H-pyrazol-4-carboxylat

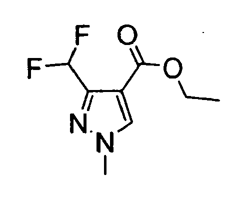

10,0 g (30 mmol) N-[(2E)-1-(Difluormethyl)-3-(dimethylamino)-2-(ethoxycarbonyl)prop-2-en-1-yliden]-N-methylmethanaminiumtetrafluoroborat wurden in 50 ml Acetonitril gelöst und mit 2,3 g Methylhydrazin versetzt. Nach 2 h Rühren bei RT wurde das Acetonitril vollständig im Vakuum entfernt. Durch Destillation im Vakuum oder Kristallisation aus n-Hexan wurden 5,3 g (86 %) des Produktes mit einem Smp. von 63-65 °C erhalten.

**¹⁹F-NMR** (CDCl₃): δ =-117,2 (d) ppm.

¹H-NMR (CDCl₃): δ= 1,35 (t, 3H); 3,96 (s, 3H); 4,31 (qu, 2H); 7,10 (t, 1H), 8,15 (s, 1H) ppm.

### Beispiel 4:

### Ethyl 3-(chlorfluormethyl)-1-methyl-1H-pyrazol-4-carboxylat

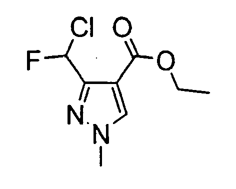

Abweichend von Beispiel 3 wird N-[(2E)-1-(Difluormethyl)-3-(dimethylamino)-2-(ethoxycarbonyl)prop-2-en-1-yliden]-N-methylmethanaminiumtetrafluoroborat eingesetzt.

**¹⁹F-NMR** (CDCl₃): δ=-133,8 (d, J=47,5) ppm.

### Beispiel 5:

### Ethyl-3-(1,2,2,2-tetrafluoroethyl)-1-methyl-1H-pyrazol-4-carboxylat

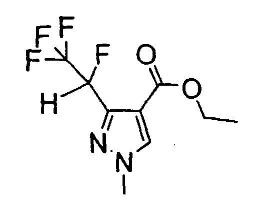

25.3 g (0,1 mmol) N,N-diethyl-1,1,2,3,3,3-hexafluoropropylamin wurden in 100 ml Dichlormethan unter Argon vorgelegt und bei RT mit 13,6 g (0,1 mol) Bortrifluoriddiethylether Komplex versetzt. Nach 30 min Rühren wurden 14,3 g (0,1 mmol) Dimethylaminoacrylsäureethylester zugegeben und 2 h bei RT gerührt. Nach dem Entfernen des Dichlormethan im Vakuum erhielt man ca 35 g des Vinamidimium Salzes. 5.6 g Methylhydrazin wurden in 40 ml Acetonitril vorgelegt und die Lösung von Vinamidinium Salz in 30 ml Acetonitril wurden bei 10 °C zugegeben.

Nach 2 h Rühren bei RT wurde das Acetonitril vollständig im Vakuum entfernt. Durch Chromatographie am SiO₂ wurden 20 g (82 %) des Produktes als Öl isoliert.

¹⁹F-NMR (CDCl₃): δ =-76.8 (dd., 3F), -191.86 (d.qw, 1F)- ppm.

¹H-NMR (CDCl₃): δ= 1,35 (t, 3H); 3,96 (s, 3H); 4,35 (qu, 2H); 6.52 (d,qu, 1H), 8,10 (s, 1H) ppm.

### Beispiel 6

### N-[(2E)-1-(difluoromethyl)-3-methoxy-2-(methoxycarbonyl)prop-2-en-1-ylidene]-N-methylmethanaminium tetrafluoroborat

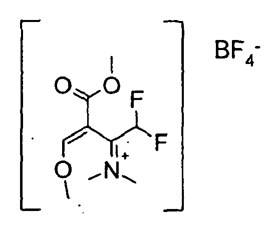

8,7 g (60 mmol) N-1,1,2,2-Tetrafluorethyldimethylamin wurden in 50 ml Dichlormethan unter Argon vorgelegt und 8,2 g (60 mmol) Bortrifluoriddiethylether Komplex bei RT zugegeben. Das Gemisch wurde 30 min gerührt und dann mit 6.38 g (55 mmol) Methoxyacrylsäuremethylesterr versetzt. Nach 2h Rühren bei RT und Entfernen des Dichlormethans im Vakuum wurden 12,4 g des Produktes (100 % Ausbeute) als gelbes Öl erhalten.

**¹⁹F-NMR** (CDCl₃) δ=-121,55, (d, 2F, J=51 Hz); -150,2 (s, 4F) ppm.

### Beispiel 7

### Methyl-3-(difluoromethyl)-1-methyl-1H-pyrazol-4-carboxylat

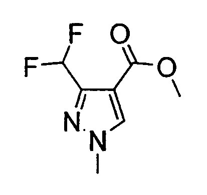

man arbeitet wie in Beispiel 2 beschrieben nimmt man jedoch N-[(2E)-1-(difluormethyl)-3-methoxy-2-(methoxycarbonyl)prop2-en-1-yliden]-N-methylmethanaminium-tetrafluoroborat

Mittels Chromatographie am Si02 isoliert man das Produkt als gelbes Öl.

**¹⁹F-NMR** (CDCl₃): δ =-117,5 (d) ppm.

### Beispiel 8

### Ethyl-3-(difluoromethyl)-1-methyl-1H-pyrazol-4-carboxylat (one-pot process)

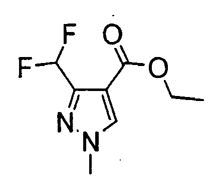

10.8 g N-1,1,2,2-Tetrafluorethyldimethylamin wurden in 50 ml Acetonitril unter Argon vorgelegt und 26 g Bortrifluorid als 17 % Lösung in CH₃CN bei RT zugegeben. Das Gemisch wurde 30 min gerührt und dann mit 8.67 g Dimethylaminoacrylsäureethylester versetzt. Das Gemisch wurde 2h bei RT gerührt und dann zu der Lösung von 3.4 g Methylhydrazin in 10 ml Acetonitril bei 10 °C langsam zugegeben.. Nach 2 h Rühren bei RT wurde das Acetonitril vollständig im Vakuum entfernt und das Produkt mit Wasser versetzt und abfiltriert. Durch Destillation im Vakuum oder waschen mit Cyclohexan wurden 10 g des Produktes mit der Reinheit von 99 % und einem Smp. von 62-63°C erhalten.

## Patentansprüche

1. Verfahren zum Herstellen von 3-Dihalogenmethyl-pyrazol-4-carbonsäurederivaten der Formel (I) 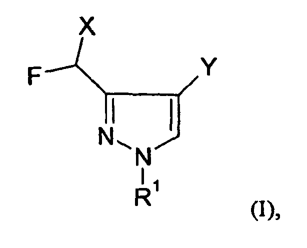 in welcher
R¹ ausgewählt ist aus C₁₋₁₂-Alkylresten, C₅₋₁₈-Aryl- oder C₇₋₁₉-Arylalkylresten,
Y ausgewählt ist aus (C=O)OR⁶, CN und (C=O)NR⁷R^{8,} wobei R⁶ R⁷ und R⁸ unab- hängig voneinander ausgewählt sind aus C₁₋₁₂-Alkylresten, C₅₋₁₈-Aryl- oder C₇₋₁₉- Arylalkylresten; und
X F, Cl oder CF₃ ist
durch Umsetzung von α-Fluoraminen der Formel (III) 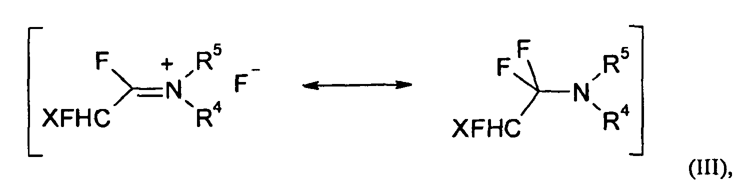 in welcher
R⁴ ausgewählt ist aus C₁₋₁₂-Alkylresten, C₅₋₁₈-Aryl- oder C₇₋₁₉-Arylalkylresten,
R⁵ unabhängig von R⁴ ausgewählt ist aus C₁₋₁₂-Alkylresten, C₅₋₁₈-Aryl- oder C₇₋₁₉- Arylalkylresten, und
X F, Cl oder CF₃ ist,
in Gegenwart von Lewissäuren (Z) mit Acrylsäurederivaten der Formel (II) 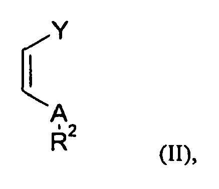 in welcher
A ausgewählt ist aus O, S und NR³,
Y ausgewählt ist aus (C=O)OR⁶, CN und (C=O)NR⁷R^{8,} wobei R⁶ R⁷ und R⁸ unabhängig voneinander ausgewählt sind aus C₁₋₁₂-Alkylresten, C₅₋₁₈-Aryl- oder C₇₋₁₉-Arylalkylresten; und
R² und R³ unabhängig voneinander ausgewählt sind aus C₁₋₁₂-Alkylresten, C₅₋₁₈-Aryl- oder C₇₋₁₉-Arylalkylresten, -OR', -SR', -NR'₂ , wobei R' ein (C₁-C₅) Alkylrest sein kann,
oder R² und R³ gemeinsam mit dem N-Atom, an das sie gebunden sind einen fünf- oder sechsgliedrigen Ring bilden können,
zu Vinamidiniumsalzen der Formel (IV) 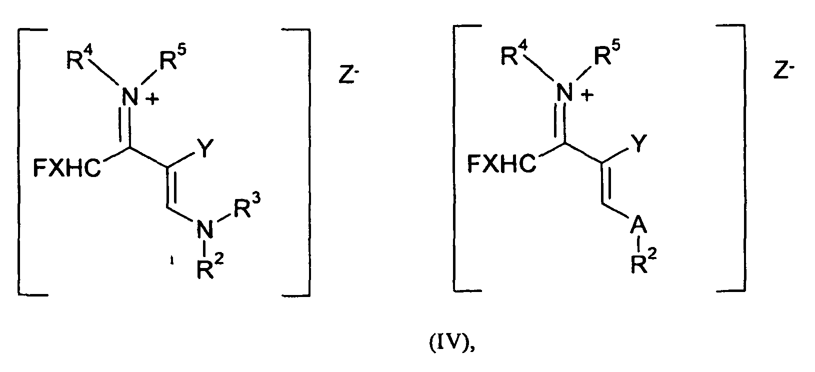 in welcher Z⁻ ein Anion ist;
und deren anschließender Umsetzung mit Alkylhydrazinen gemäß der allgemeinen Formel (V)

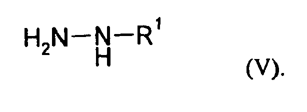

2. Verfahren zum Herstellen von 3-Dihalogenmethyl-pyrazol-4-carbonsäurederivaten der Formel (I) 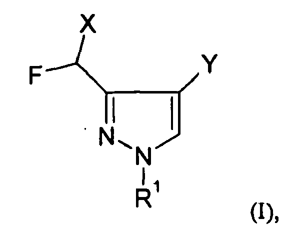 in welcher
R¹ ausgewählt ist aus Wasserstoff, C₁₋₁₂-Alkylresten, C₅₋₁₈-Aryl- oder C₇₋₁₉-Arylalkyl- resten,
Y ausgewählt ist aus (C=O)OR⁶, CN und (C=O)NR⁷R^{8,} wobei R⁶ R⁷ und R⁸ unab- hängig voneinander ausgewählt sind aus C₁₋₁₂-Alkylresten, C₅₋₁₈-Aryl- oder C₇₋₁₉- Arylalkylresten; und
X F, Cl oder CF₃ ist
durch Umsetzung von α-Fluoraminen der Formel (III) 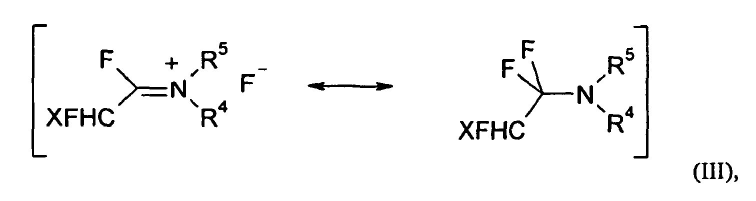 in welcher
R⁴ ausgewählt ist aus C₁₋₁₂-Alkylresten, C₅₋₁₈-Aryl- oder C₇₋₁₉-Arytalkylresten,
R⁵ unabhängig von R⁴ ausgewählt ist aus C₁₋₁₂-Alkylresten, C₅₋₁₈-Aryl- oder C₇₋₁₉- Arylalkylresten, und
X F, Cl oder CF₃ ist,
mit Acrylsäurederivaten der Formel (II) 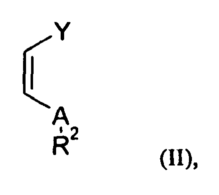 in welcher
A ausgewählt ist aus O, S und NR³,
Y ausgewählt ist aus (C=O)OR⁶, CN und (C=O)NR⁷R^{8,} wobei R⁶ R⁷ und R⁸ unabhängig voneinander ausgewählt sind aus C₁₋₁₂-Alkylresten, C₅₋₁₈-Aryl- oder C₇₋₁₉-Arylalkylresten; und
R² und R³ unabhängig voneinander ausgewählt sind aus C₁₋₁₂-Alkylresten, C₅₋₁₈-Aryl- oder C₇₋₁₉-Arylalkylresten, -OR', -SR', -NR'₂ , wobei R' ein (C₁-C₅) Alkylrest sein kann,
oder R² und R³ gemeinsam mit dem N-Atom, an das sie gebunden sind einen fünf- oder sechsgliedrigen Ring bilden können,
und deren anschließender Umsetzung mit Hydrazinen gemäß der allgemeinen Formel (V)

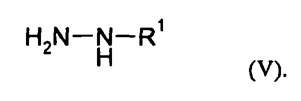

3. Verfahren gemäß Ansprüchen 1 oder 2, wobei das Anion Z⁻ ausgewählt ist aus der Gruppe bestehend aus [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

4. Verfahren gemäß einem der Ansprüche 1 bis 3, **dadurch gekennzeichnet, dass** die Gruppe Y eine Carbonsäureestergruppe der Formel (C=O)OR⁶ ist, wobei R⁶ ausgewählt ist aus C₁₋₁₂-Alkylresten, C₅₋₁₈-Aryl- oder C₇₋₁₉-Arylalkylresten.

5. Verfahren gemäß einem der Ansprüche 1 bis 4, **dadurch gekennzeichnet, dass** alle Reaktionsschritte ohne zwischenzeitliche Aufreinigung/Isolation der Zwischenprodukten durchgeführt werden.

6. Vinamidiniumsalz der Formel (IV) 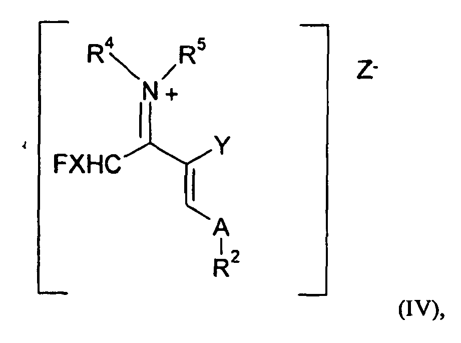 in welcher
alle Reste die zuvor definierten Bedeutungen haben und
Z⁻ ein Anion ist.

7. Vinamidiniumsalz gemäß Anspruch 6, wobei X = F ist.

8. Vinamidiniumsalz gemäß einem der Ansprüche 6 oder 7, **dadurch gekennzeichnet, dass** die Gruppe Y eine Carbonsäureestergruppe der Formel (C=O)OR⁶ ist, wobei R⁶ ausgewählt ist aus C₁₋₁₂-Alkylrestän, C₅₋₁₈-Aryl- oder C₇₋₁₉-Arylalkylresten.

9. Vinamidiniumsalz gemäß einem der Ansprüche 6 bis 8, **dadurch gekennzeichnet, dass** das Anion Z- ausgewählt ist aus der Gruppe bestehend aus [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

10. Verbindung der Formel (VI)

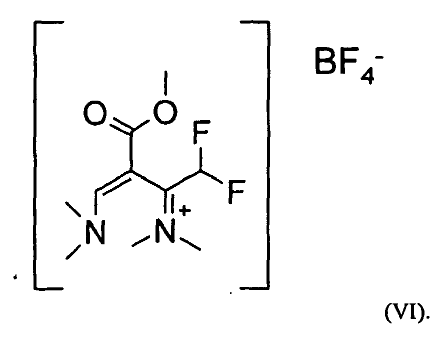

## Claims

1. Process for preparing 3-dihalomethylpyrazole-4-carboxylic acid derivatives of the formula (I) 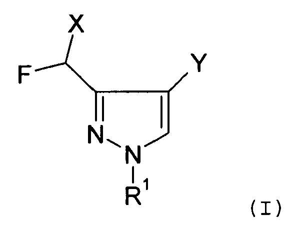 in which
R¹ is selected from C₁₋₁₂-alkyl radicals, C₅₋₁₈-aryl or C₇₋₁₉-arylalkyl radicals,
Y is selected from (C=O) OR⁶, CN and (C=O)NR⁷R⁸, where R⁶, R⁷ and R⁸ are each independently selected from C₁₋₁₂-alkyl radicals, C₅₋₁₈-aryl or C₇₋₁₉-arylalkyl radicals; and
X is F, Cl or CF₃
by reacting α-fluoroamines of the formula (III) 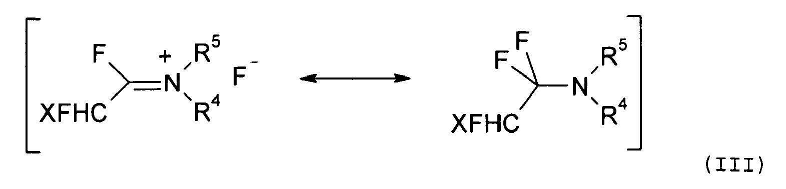 in which
R⁴ is selected from C₁₋₁₂-alkyl radicals, C₅₋₁₈-aryl or C₇₋₁₉-arylalkyl radicals,
R⁵, independently of R⁴, is selected from C₁₋₁₂-alkyl radicals, C₅₋₁₈-aryl or C₇₋₁₉-arylalkyl radicals, and
X is F, Cl or CF₃,
in the presence of Lewis acids (Z) with acrylic acid derivatives of the formula (II) 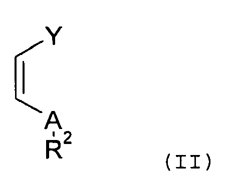 in which
A is selected from O, S and NR³,
Y is selected from (C=O)OR⁶, CN and (C=O)NR⁷R⁸, where R⁶, R⁷ and R⁸ are each independently selected from C₁₋₁₂-alkyl radicals, C₅₋₁₈-aryl or C₇₋₁₉-arylalkyl radicals; and
R² and R³ are each independently selected from C₁₋₁₂- alkyl radicals, C₅₋₁₈-aryl or C₇₋₁₉-arylalkyl radicals, -OR', -SR', -NR'₂, where R' may be a (C₁-C₅) alkyl radical,
or R² and R³, together with the nitrogen atom to which they are bonded, may form a five- or six-membered ring,
to give vinamidinium salts of the formula (IV) 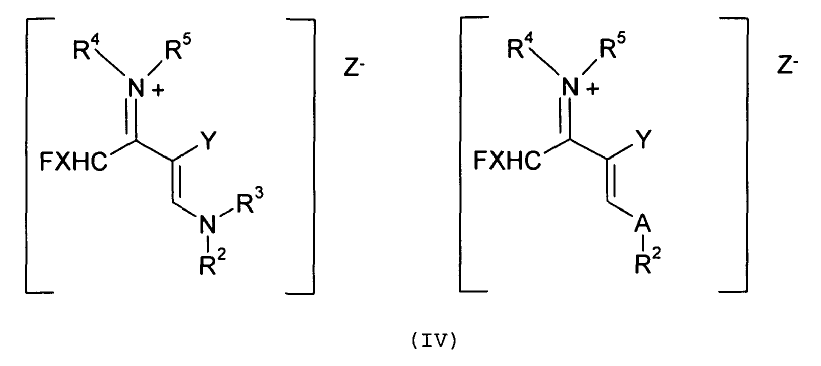 in which Z⁻ is an anion;
and the subsequent reaction thereof with alkylhydrazines of the general formula (V)

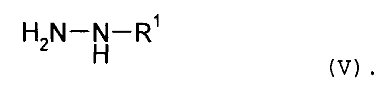

2. Process for preparing 3-dihalomethylpyrazole-4-carboxylic acid derivatives of the formula (I) 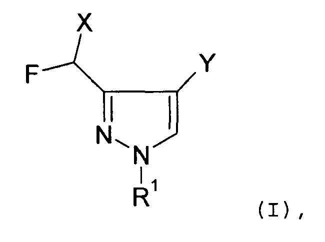 in which
R¹ is selected from hydrogen, C₁₋₁₂-alkyl radicals, C₅₋ ₁₈-aryl or C₇₋₁₉-arylalkyl radicals,
Y is selected from (C=O) OR⁶, CN and (C=O) NR⁷R⁸, where R⁶, R⁷ and R⁸ are each independently selected from C₁₋₁₂-alkyl radicals, C₅₋₁₈-aryl or C₇₋₁₉-arylalkyl radicals; and
X is F, Cl or CF₃
by reacting α-fluoroamines of the formula (III) 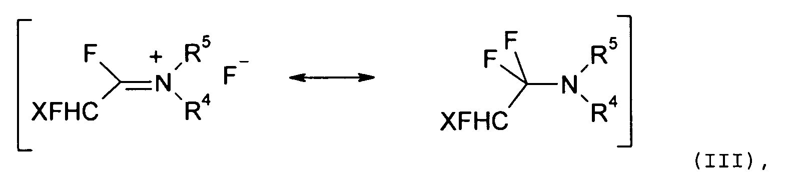 in which
R⁴ is selected from C₁₋₁₂-alkyl radicals, C₅₋₁₈-aryl or C₇₋₁₉-arylalkyl radicals,
R⁵, independently of R⁴, is selected from C₁₋₁₂-alkyl radicals, C₅₋₁₈-aryl or C₇₋₁₉-arylalkyl radicals, and
X is F, Cl or CF₃,
with acrylic acid derivatives of the formula (II) 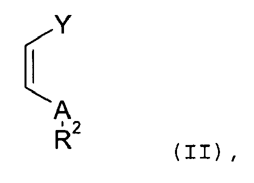 in which
A is selected from O, S and NR³,
Y is selected from (C=O)OR⁶, CN and (C=O)NR⁷R⁸, where R⁶, R⁷ and R⁸ are each independently selected from C₁₋₁₂-alkyl radicals, C₅₋₁₈-aryl or C₇₋₁₉-arylalkyl radicals; and
R² and R³ are each independently selected from C₁₋₁₂- alkyl radicals, C₅₋₁₈-aryl or C₇₋₁₉-arylalkyl radicals, -OR', -SR', -NR'₂, where R' may be a (C₁-C₅) alkyl radical,
or R² and R³, together with the nitrogen atom to which they are bonded, may form a five- or six-membered ring,
and the subsequent reaction thereof with hydrazines of the general formula (V)

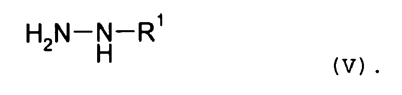

3. Process according to Claim 1 or 2, wherein the anion Z⁻ is selected from the group consisting of [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

4. Process according to one of Claims 1 to 3, **characterized in that** the Y group is a carboxylic ester group of the formula (C=O)OR⁶ where R⁶ is selected from C₁₋₁₂-alkyl radicals, C₅₋₁₈-aryl or C₇₋₁₉-arylalkyl radicals.

5. Process according to one of Claims 1 to 4, **characterized in that** all reaction steps are performed without intermediate purification/isolation of the intermediates.

6. Vinamidinium salt of the formula (IV) 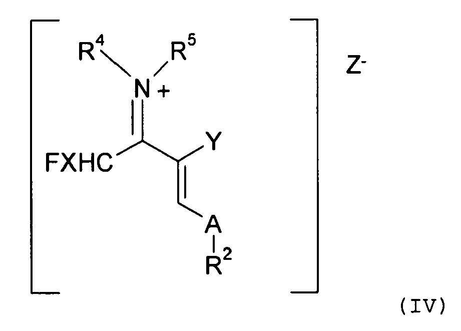 in which
all radicals are as defined above and
Z⁻ is an anion.

7. Vinamidinium salt according to Claim 6, where X = F.

8. Vinamidinium salt according to either of Claims 6 and 7, **characterized in that** the Y group is a carboxylic ester group of the formula (C=O)OR⁶ where R⁶ is selected from C₁₋₁₂-alkyl radicals, C₅₋₁₈-aryl or C₇₋₁₉-arylalkyl radicals.

9. Vinamidinium salt according to one of Claims 6 to 8, **characterized in that** the anion Z⁻ is selected from the group consisting of [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

10. Compound of the formula (VI)

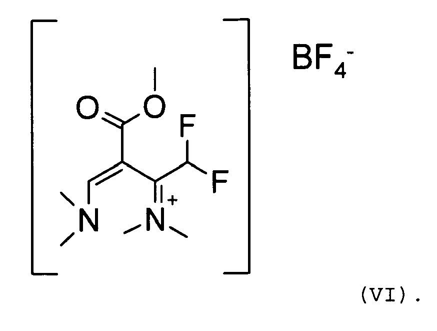

## Revendications

1. Procédé pour la préparation de dérivés de l'acide 3-dihalogénométhylpyrazole-4-carboxylique de formule (I) 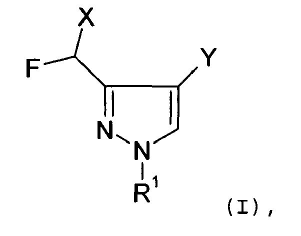 dans laquelle
R¹ est choisi parmi les radicaux C₁₋₁₂-alkyle, C₅₋₁₈- aryle ou C₇₋₁₉-arylalkyle,
Y est choisi parmi (C=O) OR⁶, CN et (C=O)NR⁷R⁸, où R⁶, R⁷ et R⁸ sont choisis, indépendamment l'un de l'autre, parmi les radicaux C₁₋₁₂-alkyle, C₅₋₁₈-aryle ou C₇-C₁₉-arylalkyle ; et
X représente F, Cl ou CF₃
par transformation d'α-fluoroamines de formule (III) 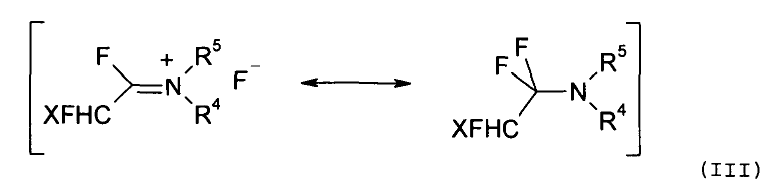 dans laquelle
R⁴ est choisi parmi les radicaux C₁₋₁₂-alkyle, C₅₋₁₈- aryle ou C₇₋₁₉-arylalkyle,
R⁵ est choisi indépendamment de R⁴ parmi les radicaux C₁₋₁₂-alkyle, C₅₋₁₈-aryle ou C₇₋₁₉-arylalkyle, et
X représente F, Cl ou CF₃
en présence d'acides de Lewis (Z) avec des dérivés d'acide acrylique de formule (II) 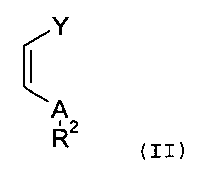 dans laquelle
A est choisi parmi O, S et NR³,
Y est choisi parmi (C=O)OR⁶, CN et (C=O) NR⁷R⁸, où R⁶, R⁷ et R⁸ sont choisis, indépendamment l'un de l'autre, parmi les radicaux C₁₋₁₂-alkyle, C₅₋₁₈-aryle ou C₇₋₁₉-arylalkyle ; et
R² et R³ sont choisis, indépendamment l'un de l'autre, parmi les radicaux C₁₋₁₂-alkyle, C₅₋₁₈-aryle ou C₇₋₁₉- arylalkyle, -OR', -SR', -NR'₂, où R' peut représenter un radical (C₁-C₅)-alkyle,
ou R² et R³, ensemble avec l'atome de N auquel ils sont liés, peuvent former un cycle de cinq ou six chaînons, en sels de vinamidinium de formule (IV) 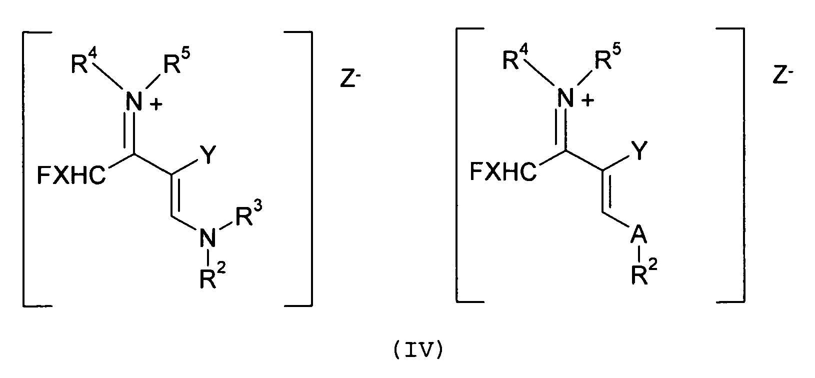 dans laquelle Z⁻ représente un anion ;
et leur transformation consécutive avec des alkylhydrazines selon la formule générale (V)

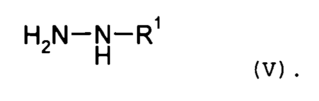

2. Procédé pour la préparation de dérivés de l'acide 3-dihalogénométhylpyrazole-4-carboxylique de formule (I) 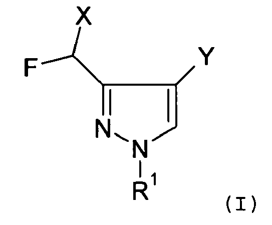 dans laquelle
R¹ est choisi parmi hydrogène, les radicaux C₁₋₁₂- alkyle, C₅₋₁₈-aryle ou C₇₋₁₉-arylalkyle,
Y est choisi parmi (C=O)OR⁶, CN et (C=O)NR⁷R⁸, où R⁶, R⁷ et R⁸ sont choisis, indépendamment l'un de l'autre, parmi les radicaux C₁₋₁₂-alkyle, C₅₋₁₈-aryle ou C₇₋₁₉-arylalkyle ; et
X représente F, Cl ou CF₃
par transformation d'α-fluoroamines de formule (III) 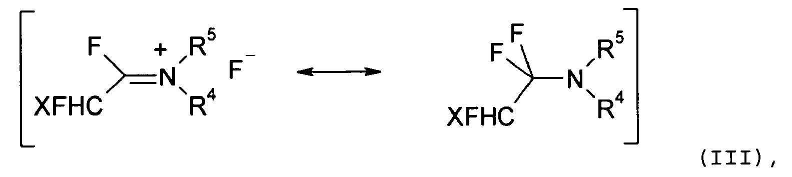 dans laquelle
R⁴ est choisi parmi les radicaux C₁₋₁₂-alkyle, C₅₋₁₈- aryle ou C₇₋₁₉-arylalkyle,
R⁵ est choisi indépendamment de R⁴ parmi les radicaux C₁₋₁₂-alkyle, C₅₋₁₈-aryle ou C₇₋₁₉-arylalkyle, et
X représente F, Cl ou CF₃
avec des dérivés d'acide acrylique de formule (II) 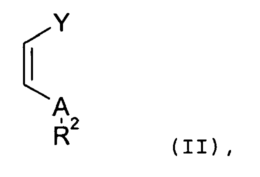 dans laquelle
A est choisi parmi O, S et NR³,
Y est choisi parmi (C=O) OR⁶, CN et (C=O) NR⁷R⁸, où R⁶, R⁷ et R⁸ sont choisis, indépendamment l'un de l'autre, parmi les radicaux C₁₋₁₂-alkyle, C₅₋₁₈-aryle ou C₇₋₁₉-arylalkyle ; et
R² et R³ sont choisis, indépendamment l'un de l'autre, parmi les radicaux C₁₋₁₂-alkyle, C₅₋₁₈-aryle ou C₇₋₁₉- arylalkyle, -OR', -SR', -NR'₂, où R' peut représenter un radical (C₁-C₅)-alkyle,
ou R² et R³, ensemble avec l'atome de N auquel ils sont liés, peuvent former un cycle de cinq ou six chaînons, et leur transformation consécutive avec des hydrazines selon la formule générale (V)

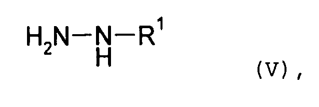

3. Procédé selon les revendications 1 ou 2, où l'anion Z⁻ est choisi dans le groupe constitué par [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

4. Procédé selon l'une quelconque des revendications 1 à 3, **caractérisé en ce que** le groupe Y est un groupe ester d'acide carboxylique de formule (C=O) OR⁶, où R⁶ est choisi parmi les radicaux C₁₋₁₂-alkyle, C₅₋₁₈-aryle ou C₇₋₁₉-arylalkyle.

5. Procédé selon l'une quelconque des revendications 1 à 4, **caractérisé en ce que** toutes les étapes de réaction sont réalisées sans purification/isolement intermédiaire des produits intermédiaires.

6. Sel de vinamidinium de formule (IV) 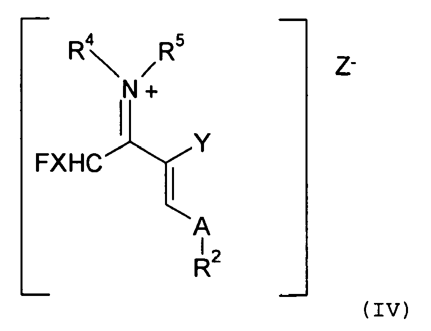 dans laquelle
tous les radicaux présentent les significations définies ci-dessus et
Z⁻ représente un anion.

7. Sel de vinamidinium selon la revendication 6, où X = F.

8. Sel de vinamidinium selon l'une quelconque des revendications 6 ou 7, **caractérisé en ce que** le groupe Y est un groupe ester d'acide carboxylique de formule (C=O)OR⁶, où R⁶ est choisi parmi les radicaux C₁₋₁₂-alkyle, C₅₋₁₈-aryle ou C₇₋₁₉-arylalkyle.

9. Sel de vinamidinium selon l'une quelconque des revendications 6 à 8, **caractérisé en ce que** l'anion Z⁻ est choisi dans le groupe constitué par [BF₄]⁻, [AlCl₄]⁻, [AlF₄]⁻, [ZnCl₃]⁻, [PF₆]⁻, [SbF₆]⁻, [SnCl₅]⁻, [BiCl₄]⁻, [GaCl₄]⁻.

10. Composé de formule (VI)
